# EUROPÄISCHE PATENTSCHRIFT

(11) **EP 0 821 233 B1**
(45) Veröffentlichungstag und Bekanntmachung des Hinweises auf die Patenterteilung: **25.09.2002**
(21) Anmeldenummer: 97112360.9
(22) Anmeldetag: 18.07.1997
(51) Int. Cl.: G01N 33/52

(54) **Volumenunabhängiger diagnostischer Testträger und Verfahren zur Bestimmung von Analyten mit dessen Hilfe**
Volume independent diagnostic test element and method to assay analytes using it
Moyen diagnostique indépendant du volume et méthode de détermination d'analytes l'utilisant

(30) Priorität: 23.07.1996 DE 19629657
(43) Veröffentlichungstag der Anmeldung: 28.01.1998
(73) Patentinhaber: Roche Diagnostics GmbH, 68298 Mannheim (DE)
(72) Erfinder: Thym, Detlef, Dr., 68259 Mannheim (DE); Leininger, Helmut, 68259 Mannheim (DE)

(56) Entgegenhaltungen:
- EP-A- 0 064 710
- EP-A- 0 348 006
- EP-A- 0 388 782
- EP-A- 0 535 480
- US-A- 2 785 057

## Beschreibung

Die Erfindung betrifft einen diagnostischen Testträger enthaltend eine Tragschicht mit einer oder mehreren darauf angeordneten zur Bestimmung von Analyt in flüssiger Probe erforderliche Reagenzien enthaltenden Nachweisschichten und ein die Nachweisschichten überdeckendes Netzwerk, das größer als die Nachweisschichten ist und das auf der Tragschicht befestigt ist. Die Erfindung betrifft außerdem die Verwendung dieses diagnostischen Testträgers zur Bestimmung von Analyt in einer Flüssigkeit und ein Verfahren zur Bestimmung von Analyt in flüssiger Probe mit Hilfe eines erfindungsgemäßen diagnostischen Testträgers.

Zur qualitativen oder quantitativen analytischen Bestimmung von Bestandteilen von Körperflüssigkeiten, insbesondere von Blut, werden oft sogenannte trägergebundene Tests verwendet. Bei diesen liegen Reagenzien auf oder in entsprechenden Schichten eines festen Testträgers vor, der mit der Probe in Kontakt gebracht wird. Die Reaktion von flüssiger Probe und Reagenzien führt zu einem nachweisbaren Signal, insbesondere einer Farbänderung, welche visuell oder mit Hilfe eines Geräts, meistens reflektionsphotometrisch, ausgewertet werden kann.

Testträger sind häufig als Teststreifen ausgebildet, die im wesentlichen aus einer länglichen Tragschicht aus Kunststoffmaterial und darauf angebrachten Nachweisschichten als Testfelder bestehen. Es sind jedoch auch Testträger bekannt, die als quadratische oder rechteckige Plättchen gestaltet sind.

Testträger der eingangs bezeichneten Art sind beispielsweise aus der deutschen Patentschrift 21 18 455 bekannt. Dort werden diagnostische Testträger zum Nachweis von Analyten in Flüssigkeiten beschrieben, die aus einer Tragschicht und mindestens einer die Nachweisreagenzien enthaltenden Nachweisschicht, deren nicht auf der Tragschicht anliegende Oberfläche mit einer Deckschicht versehen ist, bestehen. Die Deckschicht kann aus einem feinmaschigen Netzwerk in Form eines Gewebes, Gewirkes oder Vlieses bestehen. Kunststoffgewebe werden als bevorzugte Netzwerke angegeben, um eine schnelle Benetzung der Nachweisschicht mit Probenflüssigkeit zu erreichen und störende Chromatographieeffekte zu venneiden. Zum Nachweis eines Analyts in einer Flüssigkeit wird ein solcher diagnostischer Testträger in eine entsprechende Flüssigkeit, vorzugsweise Urin, eingetaucht. Die Nachweisschicht kommt so mit einem sehr großen Überschuß an Flüssigkeit in Kontakt, der nicht von dem Testträger aufgenommen werden kann. Je nach der Dauer des Kontaktes der Nachweisschicht mit der zu untersuchenden Flüssigkeit können jedoch unterschiedliche Farbintensitäten beobachtet werden. Längere Kontaktzeiten führen in der Regel zu um so positiveren Resultaten. Eine korrekte quantitative Analytbestimmung ist deshalb so nicht möglich.

Auch in EP-A 0 064 710, EP-A 0 388 782 und US 2,784,057 werden ähnliche Testelemente beschrieben, wobei in US 2,784,057 auch Testelemente mit mehreren, nebeneinanderliegenden Testfeldern für unterschiedliche Analyten offenbart sind.

Eine häufige Ursache für falsche Meßwerte beim Diabetes-Monitoring, das heißt, der regelmäßigen Kontrolle des Blutes Diabeteskranker auf den Gehalt an Glucose, ist andererseits ein zu geringes Probenvolumen. Testträger mit möglichst geringem Volumenbedarf sind deshalb das Ziel vielfältiger derzeitiger Entwicklungen. Solche Testträger müssen jedoch nicht nur mit sehr kleinen Probenvolumina von etwa 3µl richtige Meßwerte ergeben, sondern sie müssen auch bei relativ großen Probenvolumina von etwa 15-20 µl zuverlässig arbeiten und müssen die Probenflüssigkeit halten. Im Falle eines Herauslaufens von Flüssigkeit aus dem Testträger können nämlich hygienische Probleme auftreten, beispielsweise dann, wenn potentiell infektiöses Fremdblut vermessen wird oder wenn der Testträger apparativ vermessen werden soll und dann die Gefahr der Verschmutzung des Meßgerätes besteht. Dieses Ziel ist nach Kenntnis des Patentanmelders bisher noch nicht in zufriedenstellender Weise auf einfache Art erreicht worden.

Aufgabe der vorliegenden Erfindung ist es deshalb, einen diagnostischen Testträger zur quantitativen Bestimmung von Analyt in einer Flüssigkeit zur Verfügung zu stellen, auf den eine undosierte Probenflüssigkeitsmenge aufgegeben werden kann. Probenvolumina ab 3µl sollen ausreichen. Ein Probenflüssigkeitsüberschuß soll jedoch nicht zu zeitabhängigen falsch-positiven Resultaten führen. Außerdem soll überschüssige Probenflüssigkeit keine hygienischen Probleme bereiten und der Testträger soll möglichst einfach herzustellen sein.

Diese Aufgabe wird durch die in den Patentansprüchen näher charakterisierte Erfindung gelöst.

Gegenstand der Erfindung ist nämlich ein diagnostischer Testtrager mit einer Tragschicht und einer darauf angeordneten Nachweisschicht, welche zur Bestimmung von Analyt in flüssiger Probe erforderliche Reagenzien enthält. Die Nachweisschicht wird von einem Netzwerk überdeckt, das größer als die Nachweisschicht ist und das außerhalb der Nachweisschicht auf der Tragschicht befestigt ist. Das Netzwerk des erfindungsgemäßen diagnostischen Testträgers ist hydrophil, aber alleine nicht kapillaraktiv. Über den über die Nachweisschichten hinausragenden Bereichen des Netzwerkes ist eine inerte Abdeckung aus probenflüssigkeitsundurchlässigem Material so angeordnet, daß auf dem Bereich des Netzwerkes, der über einer Nachweisschicht liegt, eine Fläche zur Probenaufgabe frei bleibt.

Gegenstand der Erfindung ist außerdem die Verwendung eines solchen diagnostischen Testträgers zur Bestimmung von Analyt in einer Flüssigkeit. Insofern ist auch ein Verfahren zur Bestimmung von Analyt in flüssiger Probe mit Hilfe eines solchen diagnostischen Testträgers Gegenstand der Erfindung, bei dem Probenflüssigkeit auf die Probenauftragsstelle aufgegeben wird. Das Netzwerk führt überschüssige Flüssigkeit von der Nachweisschicht in den über die Nachweisschicht hinausragenden Bereich des Netzwerkes, woraufhin dann die Nachweisschicht auf eine Signalbildung hin beobachtet wird. Die Signalbildung ist ein Maß fiir die Anwesenheit bzw. Menge an Analyt in der untersuchten flüssigen Probe.

Das Netzwerk des erfindungsgemäßen diagnostischen Testträgers soll selbst nicht kapillaraktiv oder saugfähig sein, damit die Probenflüssigkeit möglichst vollständig fiir die Nachweisschicht zur Verfügung steht. Als geeignet haben sich solche Netzwerke erwiesen, die beim senkrechten Eintauchen in Wasser eine Steighöhe des Wassers im Netzwerk von weniger als 2 mm ermöglichen. Vorzugsweise werden als Netzwerk grobmaschige monofile Gewebe eingesetzt, die hydrophil sind. Hierfür kann das Gewebematerial selbst hydrophil sein oder es kann, beispielsweise durch Behandlung mit Netzmittel, hydrophil gemacht werden. Als besonders bevorzugtes Netzmaterial wird Polyester verwendet, wobei das Netz aus diesem Material dann mit Netzmittel behandelt eingesetzt wird.

Die Dicke des Netzwerkes muß so beschaffen sein, daß die darauf liegende Abdeckung und die darunter liegende Schicht sich in einem solchen Abstand voneinander befinden, daß verbleibende Flüssigkeit über der gesattigten Nachweisschicht und in den gefüllten Maschen des Netzwerkes durch Kapillarkraft in den Bereich unter der Abdeckung aufgesaugt und von der Probenauftragsstelle weggeführt wird. In der Regel ist hierfür eine Dicke des Netzwerkes von 50 bis 400 µm vorteilhaft.

Das Netz muß eine ausreichend große Maschenweite aufweisen, damit Flüssigkeit durch das Netz auf die Nachweisschicht gelangt. Aufgrund der Beschaffenheit des Netzwerkes wird Flüssigkeit nicht im Netz über die Netzoberfläche horizontal gespreitet, sondern sie fließt vertikal durch das Netz auf die Nachweisschicht.

In einem erfindungsgemäßen diagnostischen Testträger kommen für die Tragschicht insbesondere solche Materialien in Frage, die die zu untersuchenden Flüssigkeiten nicht aufnehmen. Dies sind sogenannte nicht-saugfähige Materialien, wobei Kunststoffolien beispielsweise aus Polystyrol, Polyvinylchlorid, Polyester, Polycarbonat oder Polyamid besonders bevorzugt sind. Es ist jedoch auch möglich, saugfähige Materialien, wie zum Beispiel Holz, Papier oder Pappe mit wasserabstoßenden Mitteln zu imprägnieren oder mit einem wasserfesten Film zu überziehen, wobei als Hydrophobierungsmittel Silikone oder Hartfette und als Filmbildner beispielsweise Nitrocellulose oder Celluloseacetat verwendet werden können. Als weitere Trägermaterialien eignen sich Metallfolien oder Glas.

Für eine Nachweisschicht ist es im Gegensatz hierzu erforderlich, solche Materialien einzusetzen, die in der Lage sind, die zu untersuchende Flüssigkeit mit darin enthaltenen Inhaltsstoffen aufzunehmen. Dies sind sogenannte saugfähige Materialien, wie beispielsweise Vliese, Gewebe, Gewirke, Membranen oder sonstige poröse Kunststoffmaterialien oder quellfähige Materialien, wie Gelantine- oder Dispersionsfilme, die als Schichtmaterialien verwendet werden können. Die für die Nachweisschicht in Frage kommenden Materialien müssen natürlich auch Reagenzien tragen können, die für den Nachweis des zu bestimmenden Analyts erforderlich sind. Im einfachsten Fall befinden sich alle für den Nachweis des Analyts erforderliche Reagenzien auf oder in einer Schicht. Es sind jedoch auch Fälle vorstellbar, fiir die es vorteilhafter ist, die Reagenzien auf mehrere saug- oder quellfähige Materialschichten zu verteilen, die dann übereinander, sich vollflächig berührend angeordnet sind. Der im folgenden verwendete Begriff "Nachweisschicht" soll sowohl solche Fälle umfassen, bei denen sich die Reagenzien entweder nur in oder auf einer Schicht oder in zwei oder noch mehr, wie vorstehend beschrieben, angeordneten Schichten befinden.

Außerdem kann die Nachweisschicht auch eine Schicht enthalten, die Plasma oder Serum aus Vollblut abzutrennen in der Lage ist, wie beispielsweise ein Glasfaservlies, wie es zum Beispiel aus EP-B-0 045 476 bekannt ist. Eine oder mehrere solcher Abtrennschichten kann über einer oder mehreren Schichten, die Nachweisreagenzien tragen, liegen. Auch ein solcher Aufbau soll von dem Begriff "Nachweisschicht" umfaßt sein.

Bevorzugte Materialien fiir die Nachweisschicht sind Papiere oder poröse Kunststoffmaterialien, wie Membranen. Hiervon besonders bevorzugt sind asymmetrisch poröse Membranen, die vorteilhafterweise so angeordnet sind, daß die zu untersuchende Probenflüssigkeit auf die großporige Seite der Membran aufgegeben wird und die Bestimmung des Analyts von der feinporigen Seite der Membran aus erfolgt. Als poröse Membranmaterialien sind Polyamid-, Polyvinylidendifluorid-, Polyethersulfon- oder Polysulfonmembranen ganz besonders bevorzugt. Hervorragend geeignet sind insbesondere Polyamid 66-Membranen und hydrophilisierte asymmetrische Polysulfonmembranen. Die Reagenzien zur Bestimmung des nachzuweisenden Analyts sind in der Regel durch Imprägnierung in die vorstehend genannten Materialien eingebracht oder durch Beschichtung einseitig aufgebracht worden. Bei Beschichtung asymmetrischer Membranen wird vorteilhafterweise die feinporige Seite beschichtet.

Für die Nachweisschicht kommen jedoch auch sogenannte offene Filme in Frage, wie sie beispielsweise in EP-B-0 016 387 beschrieben sind. Hierfür werden einer wäßrigen Dispersion von filmbildenden organischen Kunststoffen Feststoffe als feine, unlösliche, organische oder anorganische Partikel zugegeben und die für die Nachweisreaktion erforderlichen Reagenzien zusätzlich hinzugefügt. Geeignete Filmbildner sind bevorzugt organische Kunststoffe, wie Polyvinylester, Polyvinylacetate, Polyacrylester, Polymethacrylsäure, Polyacrylamide, Polyamide, Polystyrol, Mischpolymerisate, zum Beispiel von Butadien und Styrol oder von Maleinsäureester und Vinylacetat oder andere filmbildende, natürliche und synthetische organische Polymere sowie Mischungen derselben in Form von wäßrigen Dispersionen. Die Dispersionen lassen sich auf einer Unterlage zu einer gleichmäßigen Schicht verstreichen, die nach dem Trocknen einen wasserfesten Film ergibt. Die trocknen Filme haben eine Dicke von 10 µm bis 500 µm, vorzugsweise von 30 bis 200 µm. Der Film kann mit der Unterlage als Träger zusammen verwendet werden oder für die Nachweisreaktion auf einen anderen Träger aufgebracht werden. Obwohl die für die Nachweisreaktion erforderlichen Reagenzien normalerweise in die zur Herstellung der offenen Filme verwendete Dispersion gegeben werden, kann es auch vorteilhaft sein, wenn der gebildete Film nach seiner Herstellung mit den Reagenzien imprägniert wird. Auch eine Vorimprägnierung der Füllstoffe mit den Reagenzien ist möglich. Welche Reagenzien zur Bestimmung eines bestimmten Analyts eingesetzt werden können sind dem Fachmann bekannt. Dies muß hier nicht näher ausgeführt werden.

Ein weiteres Beispiel fiir eine erfindungsgemäß bevorzugte Nachweisschicht ist eine Filmschicht, wie sie in WO-A-92 15 879 beschrieben ist. Diese Schicht wird aus einer Dispersion oder Emulsion eines polymeren Filbildners hergestellt, welche zusätzlich in homogener Verteilung ein Pigment, ein Quellmittel und das Nachweisreagenz enthält. Als polymere Filmbildner eignen sich insbesondere Polyvinylester, Polyvinylacetate, Polyacrylester, Polymethacrylsäure, Polyvinylamide, Polyamide und Polystyrol. Neben Homopolymeren sind auch Mischpolymerisate, z.B. von Butadien, Styrol oder Maleinsäureester geeignet. Titandioxid ist ein für den Film besonders geeignetes Pigment. Das verwendete Quellmittel soll besonders gute Quelleigenschaften aufweisen, wobei Methylvinylethermaleinsäurenanhydrid-Copolymer besonders empfohlen wird. Welche Reagenzien zur Bestimmung eines bestimmten Analyts eingesetzt werden, bleibt dem Fachmann überlassen.

Ganz besonders bevorzugt wird in einem erfindungsgemäßen diagnostischen Testträger ein Testfeld als Nachweisschicht eingesetzt, das aus zwei Schichten aufgebaut ist. Dieses Testfeld umfaßt eine transparente Folie, auf die in dieser Reihenfolge eine erste und eine zweite Filmschicht übereinanderliegend aufgebracht sind. Wesentlich ist, daß die auf der transparenten Folie befindliche erste Schicht im feuchten Zustand bedeutend weniger lichtstreuend ist als die darüberliegende zweite Schicht. Die nicht beschichtete Seite der transparenten Folie wird als Nachweisseite bezeichnet und die Seite der zweiten Schicht, die der Seite gegenüberliegt, mit der die zweite Schicht auf der ersten aufliegt, wird als Probenaufgabenseite bezeichnet.

Die Filmschichten werden aus Dispersionen oder Emulsionen polymerer Filmbildner hergestellt. Dispersionsfilmbildner enthalten mikroskopische, in der Trägerflüssigkeit (meist Wasser) unlösliche Polymerteilchen, welche in feinster Verteilung in der Trägerflüssigkeit dispergiert sind. Wird bei der Filmbildung die Flüssigkeit durch Verdampfen entfernt, so nähern sich die Teilchen und berühren sich schließlich. Durch die dabei auftretenden großen Kräfte und einen mit der Filmbildung einhergehenden Gewinn an Oberflächenenergie wachsen die Teilchen zu einer weitgehend geschlossenen Filmschicht zusammen. Alternativ kann auch eine Emulsion des Filmbildners verwendet werden, bei der dieser in einem Lösungsmittel gelöst ist. Das gelöste Polymer ist in einer Trägerflüssigkeit emulgiert, die mit dem Lösungsmittel nicht mischbar ist.

Als Polymere für solche Filmbildner eignen sich insbesondere Polyvinylester, Polyvinylacetate, Polyacrylester, Polymethacrylsaure, Polyvinylamide, Polyamide und Polystyrol. Neben Homopolymeren sind auch Mischpolymerisate, z. B. von Butadien, Styrol oder Maleinsäureester geeignet.

In dem Testfeld befinden sich die zwei genannten Filmschichten auf einer transparenten Folie. Hierfür kommen insbesondere solche Kunststoffolien in Betracht, die flüssigkeitsundurchlässig sind. Polycarbonatfolie hat sich als besonders bevorzugt erwiesen.

Die beiden Filmschichten können aus Beschichtungsmassen hergestellt werden, die den gleichen polymeren Filmbildner enthalten oder sie können aus Beschichtungsmassen erzeugt werden, die unterschiedliche polymere Filmbildner enthalten. Während die erste Schicht ein Quellmittel und gegebenenfalls einen schwach lichtstreuenden Füllstoff enthält, benötigt die zweite Schicht ein Quellmittel und in jedem Fall wenigstens ein stark lichtstreuendes Pigment. Daneben kann die zweite Schicht auch nicht-poröse Füllstoffe sowie poröse Füllstoffe, wie Kieselgur, in geringen Mengen enthalten, ohne dadurch für Erythrozyten durchlässig zu werden.

Durch Zugabe eines gut quellenden Quellmittels (das heißt, einer Substanz, die unter Aufnahme von Wasser ihr Volumen vergrößert) erhält man nicht nur Schichten, die relativ schnell von Probenflüssigkeit penetriert werden, sondern die trotz dieser Öffnungswirkung des Quellmittels gute Erythrozyten- und außerdem auch Blutfarbstoffabtrenneigenschaften besitzen. Die Quelleigenschaften sollten so gut sein, daß fiir einen Test, bei dem die Geschwindigkeit der Farbbildung - wie beispielsweise einer Glucosenachweisreaktion - überwiegend von der Penetration der Probenflüssigkeit durch die Schicht abhängt, die optisch nachweisbare Reaktion nach maximal einer Minute meßbar ist. Als besonders geeignete Quellmittel haben sich Methylvinylethermaleinsaureanhydrid-Copolymer, Xanthangum und Methylvinylethermaleinsäure-Copolymer erwiesen.

Kieselgur wird auch als Diatomeenerde bezeichnet. Es handelt sich um aus den Kieselsäuregerüsten der Diatomeenarten entstandene Ablagerungen, die an verschiedenen Orten abgebaut werden. Die bevorzugt eingesetzte Kieselgur hat einen mittleren Teilchendurchmesser von 5-15 µm, wobei diese Werte mit einem Laser-Granulometer Typ 715 bestimmt wurden, welches von der Firma Pabisch, München, Bundesrepublik Deutschland, vertrieben wird.

Der stark lichtstreuende Pigmentanteil der zweiten Schicht liegt bei mindestens 25 Gewichts-%, bezogen auf die getrocknete und einsatzbereite Doppelschicht des Testfeldes. Da die schwach lichtstreuenden Füllstoffe und die stark lichtstreuenden Pigmente wesentlich für die optischen Eigenschaften der Filmschichten verantwortlich sind, besitzen die erste und die zweite Filmschicht unterschiedliche Füllstoffe und Pigmente.

Die erste Filmschicht soll entweder keine oder solche Füllstoffe enthalten, deren Brechungsindex nahe beim Brechungsindex von Wasser liegt. Als besonders geeignet hierfür haben sich Siliziumdioxid, Silikate und Aluminiumsilikate erwiesen. Ein Natriumaluminiumsilikat mit dem Handelsnamen Transpafill® ist besonders bevorzugt.

Die zweite Schicht soll erfindungsgemäß sehr stark lichtstreuend sein. Idealerweise liegt der Brechungsindex der Pigmente in der zweiten Filmschicht mindestens bei 2,5. Daher wird vorzugsweise Titandioxid eingesetzt. Teilchen mit einem mittleren Durchmesser von etwa 0,2 bis 0,8 µm haben sich als besonders vorteilhaft erwiesen. Leicht verarbeitbare Titandioxid-Typen in der Anatas-Modifikation sind ganz besonders bevorzugt.

Reagenzsysteme zum Nachweis bestimmter Analyte durch Farbbildung sind dem Fachmann bekannt. Es ist möglich, daß sich sämtliche Komponenten des Reagenzsystems in einer Filmschicht befinden. Es ist aber auch möglich, daß die Komponenten des Reagenzsystems auf beide Filmschichten verteilt sind. Vorteilhafterweise befindet sich das farbbildende Reagenzsystem wenigstens zum Teil in der ersten Filmschicht.

Unter Farbbildung wird im Rahmen der vorliegenden Erfindung nicht nur der Übergang von weiß nach farbig verstanden, sondern auch jede Farbveranderung, wobei natürlich solche Farbveränderungen besonders bevorzugt sind, die mit einer möglichst großen Verschiebung der maximalen Absorptionswellenlinie (λₘₐₓ) einhergehen.

Für die Optimierung des Testfeldes in dem erfindungsgemäßen diagnostischen Testträger hat es sich als besonders vorteilhaft erwiesen, wenn beide Filmschichten ein nicht hämolysierendes Netzmittel enthalten. Neutrale, das heißt, nicht geladene Netzmittel sind hierfür besonders geeignet. Ganz besonders bevorzugt wird N-Octanoyl-N-methyl-glucamid.

Zur Herstellung eines Testfeldes eines erfindungsgemäßen diagnostischen Testträgers werden die jeweiligen Filmschichten jeweils nacheinander aus einer homogenen Dispersion der genannten Bestandteile hergestellt. Hierzu verwendet man als Unterlage fiir das Ausformen der Beschichtungsmasse fiir die erste Filmschicht die transparente Folie. Nach Aufbringen der Beschichtungsmasse fiir die erste Filmschicht in einer bestimmten Schichtdicke wird die Schicht getrocknet. Danach wird auf diese Schicht die Beschichtungsmasse für die zweite Schicht ebenfalls in einer dünnen Schichtdicke aufgebracht und getrocknet. Nach dem Trocknen sollte die Dicke der ersten und zweiten Filmschicht zusammen maximal 0,2 mm, bevorzugt maximal 0,12 mm, besonders bevorzugt maximal 0,08 mm betragen. Vorzugsweise ist die trockene zweite Filmschicht etwa 2 bis 5 mal dicker als die erste.

Der erfindungsgemäße Testträger kann 1 Nachweisschicht besitzen. Er kann aber auch mehrere nebeneinander angeordnete Nachweisschichten beinhalten. Im Falle mehrerer Nachweisschichten können diese gleich oder verschieden sein, so daß ein und derselbe Analyt in mehreren Nachweisschichten nebeneinander bestimmt wird oder es können verschiedene Analyten in jeweils einer anderen Nachweisschicht nachgewiesen werden. Es ist aber auch möglich, daß sich auf einer Nachweisschicht mehrere räumlich getrennte Reaktionsbezirke nebeneinander befinden, so daß auch hier entweder der gleiche Analyt mehrmals oder verschiedene Analyten nebeneinander in derselben Nachweisschicht bestimmt werden. Im letzterem Fall ist das Material der Schicht bis auf die Reagenzien zur Bestimmung der Analyten dasselbe. In unterschiedlichen Reaktionsbezirken befinden sich unterschiedliche Reagenzien. Verschiedene Reaktionsbezirke können sich berührend nebeneinander vorliegen oder sie können durch dazwischen liegende Bereiche, die mit Analyt kein Signal bilden, getrennt sein.

In dem erfindungsgemäßen diagnostischen Testträger ist das die Nachweisschicht überdeckende Netzwerk größer als die darunter liegende Nachweisschicht. Der über die Nachweisschicht hinausragende Teil des Netzwerkes, das ist der Teil des Netzwerkes, der nicht mit der Nachweisschicht in Berührung steht, ist außerhalb der Nachweisschicht auf der Tragschicht direkt oder indirekt über Abstandshalter befestigt. Die Befestigung kann nach dem Fachmann aus der Testträgertechnologie bekannten Methoden erfolgen. Beispielsweise kann die Befestigung mittels Schmelzkleber oder härtendem Kaltkleber erfolgen. Dabei ist eine punktuelle oder gerasterte Verklebung vorteilhaft, da der kapillaraktive Flüssigkeitstransport in diesem Fall besonders gut möglich ist. Als vorteilhaft haben sich auch doppelseitig klebende Streifen erwiesen. In allen Fällen ist es jedoch wichtig, daß die Befestigung des Netzwerkes auf der Tragschicht so erfolgt, daß von der Nachweisschicht ein kapillaraktiver Flüssigkeitstransport in den Teil des Netzwerkes möglich ist, der auf der Tragschicht befestigt ist. Dieser kapillaraktive Flüssigkeitstransport muß insbesondere dann möglich sein, wenn die Nachweisschicht mit Flüssigkeit gesättigt ist. Für die Verarbeitung ganz besonders geeignet haben sich Klebebänder mit Natur- oder Synthesekautschuk erwiesen. Ganz besonders vorteilhaft ist es, wenn das Mittel, das zur Befestigung des Netzwerkes auf der Tragschicht dient, etwa die gleiche Dicke wie die Nachweisschicht(en) hat. Es dient dann quasi als Abstandshalter, um das Netzwerk auch außerhalb des Bereiches der Nachweisschicht(en) insgesamt auf einer durchgehenden Fläche eben zu halten.

Enthält der erfindungsgemäße diagnostische Testträger mehrere Nachweisschichten nebeneinander, so kann ein Netzwerk alle Nachweisschichten überdecken oder es können mehrere Netzwerke verwendet werden.

Zur Bestimmung des in der Probenflüssigkeit nachzuweisenden Analyts ist in dem erfindungsgemäßen diagnostischen Testträger die Nachweisschicht, zumindest aber sind die Reaktionsbezirke, das heißt, Reagenz tragende Bereiche der Nachweisschicht(en), die auf Signalbildung hin beobachtet und vermessen werden können, durch die Tragschicht sichtbar. Dies kann dadurch erreicht werden, daß die Tragschicht transparent ist. Es ist aber auch möglich, daß die Tragschicht eine Lochung aufweist, die von der Nachweisschicht oder den Nachweisschichten überdeckt ist. Durch die Lochung ist dann die Nachweisschicht oder sind die Nachweisschichten zumindest aber die Reaktionsbezirke der Nachweisschichten sichtbar. In einer bevorzugten Ausführungsform des erfindungsgemäßen diagnostischen Testträgers befindet sich in der Tragschicht unterhalb einer Nachweisschicht ein Loch, durch das die Nachweisschicht oder ein Reaktionsbezirk beobachtbar ist. Das Loch besitzt einen etwas kleineren Durchmesser als die kleinste Längenausdehnung der Nachweisschicht, so daß die Nachweisschicht außerhalb des Loches auf der Tragschicht aufliegt und dort befestigt sein kann. Vorteilhafterweise ist die Nachweisschicht durch beidseitig daneben angeordnete doppelseitige Klebebänder und das über der Nachweisschicht liegende Netzwerk und dessen Befestigung auf der Tragschicht ausreichend fixiert. Bevorzugt ist jedoch die Nachweisschicht selbst auch mittels dünnem Klebeband auf der Tragschicht befestigt.

Durch ein Loch können aber auch mehrere Reaktionsbezirke einer Nachweisschicht sichtbar sein.

Die Lochung eines erfindungsgemäßen diagnostischen Testträgers kann auch aus zwei oder mehr Löchern bestehen, die zur Bestimmung von Analyt (einem oder mehreren Analyten) genutzt werden können. Über den Löchern können verschiedene Nachweisschichten angeordnet sein oder auch nur eine Nachweisschicht mit mehreren Reaktionsbezirken, so daß durch je 1 Loch eine Nachweisschicht oder je ein Reaktionsbezirk beobachtet werden kann. Es ist auch möglich, daß durch ein Loch mehrere Reaktionsbezirke beobachtet werden können.

Über dem Netzwerk des erfindungsgemäßen diagnostischen Testträgers ist eine inerte Abdeckung aus probenundurchlässigem, in der Regel wasserundurchlässigem und nicht saugfähigem Material, so angeordnet, daß der Bereich des Netzwerkes außerhalb der Nachweisschicht abgedeckt ist. Idealerweise ragt die Abdeckung auch noch ein wenig über den Bereich der Nachweisschicht. Auf jeden Fall bleibt jedoch ein erheblicher Teil des Netzwerkes, das die Nachweisschicht bedeckt, frei. Dieser freie Teil des Netzwerkes wird als Probenauftragsstelle bezeichnet.

Als Abdeckung haben sich Kunststoffolien als besonders vorteilhaft erwiesen. Wenn Abdeckung und Netzwerk unterschiedliche Farben haben, beispielsweise weiß und gelb oder weiß und rot, kann damit die Stelle sehr gut kenntlich gemacht werden, auf die zu untersuchende Probenflüssigkeit aufgegeben werden soll.

Auf der Abdeckung kann auch beispielsweise mit einem oder mehreren aufgedruckten Pfeilen verdeutlicht werden, in welcher Richtung, das heißt, mit welchem Ende ein erfindungsgemäßer diagnostischer Testträger in ein Meßgerät gelegt oder geschoben werden soll.

Eine Probenauftragsstelle kann besonders einfach durch eine Abdeckung mittels zweier bandförmiger Kunststoffolien erreicht werden, die einen bandartigen Bereich des die Nachweisschicht überdeckenden Netzwerkes frei lassen. Wenn 2 oder mehr Probenauftragsstellen vorgesehen werden, sind 3 oder mehr bandförmige Kunststoffolien zu verwenden. Die zur Abdeckung verwendeten Folien sind auf dem Netzwerk und gegebenenfalls auf der Tragschicht befestigt. Für eine solche Befestigung eignen sich Schmelzkleber, die vorzugsweise punktuell oder gerastert auf der Tragschicht oder der Unterseite der Abdeckung aufgebracht sind oder Klebebänder, wenn die Folien nicht selbst klebefähig sind. Auf jeden Fall ist jedoch darauf zu achten, daß unter der Abdeckung, durch das Netzwerk gebildet, ein Kapillarspalt verbleibt, in den überschüssige Probenflüssigkeit von einer mit Flüssigkeit gesättigten Nachweisschicht aufgenommen werden kann. Die Probenauftragsstelle befindet sich vorzugsweise über der Lochung in der Tragschicht, durch die eine Signalbildung in der Nachweisschicht beobachtet werden kann.

Zur Durchführung eines Verfahrens zur Bestimmung von Analyt in flüssiger Probe mit Hilfe eines erfindungsgemäßen diagnostischen Testträgers wird Probenflüssigkeit auf die der Nachweisschicht abgewandten Seite des Netzwerkes aufgegeben, idealerweise so viel, daß die durch das Netzwerk gelangende Flüssigkeit die Nachweisschicht vollständig sättigt. Als Probenflüssigkeit kommen insbesondere Körperflüssigkeiten wie Blut, Plasma, Serum, Urin, Speichel etc. in Frage. Blut oder von Blut abgeleitete Flüssigkeiten wie Plasma oder Serum sowie Urin sind besonders bevorzugte Probenflüssigkeiten. Überschüssige Flüssigkeit wird durch das Netzwerk von der Nachweisschicht in den über die Nachweisschicht hinausragenden Bereich des Netzwerkes weggeführt. In der Nachweisschicht kann dann bei Anwesenheit des zu bestimmenden Analyts ein Signal nachgewiesen werden. Vorteilhafterweise handelt es sich bei einem solchen Signal um eine Farbänderung, worunter sowohl Farbbildung, Farbverlust als auch Farbumschlag verstanden wird. Die Intensität der Farbänderung ist ein Maß für die Menge an Analyt in der untersuchten flüssigen Probe. Sie kann visuell oder mit Hilfe eines Gerätes, meistens reflektionsphotometrisch quantitativ ausgewertet werden.

Wenn zu wenig Flüssigkeit auf die Nachweisschicht gelangt, das heißt, weniger als zur Sättigung der Schicht erforderlich ist, bleiben von oben und unten sichtbare Bereiche der Nachweisschicht trocken, weil durch das Netzwerk Flüssigkeit nur vertikal auf die Nachweisschicht gelangt und keine horizontale Spreitung von Flüssigkeit über die Oberfläche des Netzwerkes erfolgt. Da bei Anwesenheit von Analyt eine Signalbildung nur im durchfeuchteten Bereich der Nachweisschicht erfolgt, ist sowohl durch das Netzwerk als auch durch die Tragschicht eine inhomogene Signalbildung visuell oder apparativ erkennbar. Dies ist für den die Untersuchung Durchführenden ein deutliches Zeichen dafür, daß zuwenig Probenflüssigkeit verwendet wurde und damit das Ergebnis der Untersuchung falsch sein kann. Auch wenn kein Analyt in der Probe vorhanden ist, ist in einem solchen Fall beispielsweise visuell oder durch reflektrometrische Vermessung von mehreren Teilbereichen der Nachweisschicht festzustellen, daß nur ein Teil der Nachweisschicht befeuchtet ist und somit zu wenig Probenflüssigkeit aufgegeben wurde.

Neben der Markierung der Probenauftragsstelle unterstützt eine solche Abdeckung auch noch die Kapillarkräfte, die ein Wegleiten überschüssiger Flüssigkeit von der Nachweisschicht bewirken. Außerdem führt die Abdeckung dazu, daß von der Nachweisschicht weggeleitete überschüssige Flüssigkeit von Kontakt von außen geschützt ist und solche Flüssigkeit nicht leicht von dem Testträger herabtropfen kann.

Ein großer Vorteil des erfindungsgemäßen diagnostischen Testträgers besteht darin, daß kein vorbestimmtes Volumen einer Probenflüssigkeit auf den Testträger aufgegeben werden muß. Überschüssige Flüssigkeit wird, wie bereits vorstehend erwähnt, durch das über die Nachweisschicht hinausragende Netzwerk von der Nachweisschicht weggeleitet. Weil überschüssige Flüssigkeit von der Nachweisschicht weggeleitet wird, wird auch hygienischen Gesichtspunkten Rechnung getragen. Ein Herabtropfen von Flüssigkeit aus dem Testträger oder ein Kontakt von Flüssigkeit beispielsweise mit Teilen eines Gerätes, in das der Testträger zur apparativen Auswertung gelegt wird, wird zuverlässig vermieden. Dies ist insbesondere bei der Untersuchung von Blut oder von Blut abgeleiteten Proben, wie Plasma oder Serum, ein sehr wichtiger Aspekt.

Die Größe des über die Nachweisschicht hinausragenden Bereiches des Netzwerkes (des die Nachweisschicht "überragenden" Teils des Netzwerkes) richtet sich nach dem in der Praxis größten zu erwartenden Probenvolumen, damit auch tatsächlich überschüssige Flüssigkeit von der Nachweisschicht weggeführt werden kann. Auf diese Art und Weise ist die bei Anwesenheit eines Analyts sich einstellende Signalintensität unabhängig von der Menge und der Dauer des Kontaktes der Probenflüssigkeit mit der Nachweisschicht. Die Farbe, die sich nach Beendung der Nachweisreaktion, üblicherweise innerhalb weniger Sekunden bis weniger Minuten eingestellt hat, bleibt so für die Messung unverändert. Sie wird lediglich durch die Stabilität des farbgebenden Systems bestimmt, nicht aber beispielsweise durch Analyt, der aus überschüssiger Flüssigkeit in die Nachweisschicht nachdiffundiert. Falsch positive Resultate werden so ebenfalls vermieden und eine quantitative Analytbestimmung ermöglicht.

Durch die Abdeckung von Teilen des Netzwerkes und damit der Markierung der Probenauftragsstelle wird dafür Sorge getragen, daß Flüssigkeit nur an der dafür optimalen Stelle auf die Nachweisschicht gelangen kann. In Kombination mit einer Nachweisschicht, die nur wenig Flüssigkeit aufnimmt und dennoch eine intensive Signalbildung gewährleistet, wird sichergestellt, daß bereits bei sehr kleinen Probenvolumina zuverlässige Analytbestimmungen möglich sind. Dadurch, daß der erfindungsgemäße Testträger aus nur wenigen Komponenten besteht, die einfach und schnell zusammenfügbar sind, ist er sehr preiswert herzustellen.

Bevorzugte Ausführungsformen des erfindungsgemäßen diagnostischen Testträgers sind in Fig. 1-23 dargestellt.

Fig. 1 zeigt eine perspektivische Ansicht eines erfindungsgemäßen diagnostischen Testträgers mit einer Probenauftragsstelle.

Fig. 2 zeigt eine Aufsicht auf die Unterseite eines erfindungsgemäßen diagnostischen Testträgers gemäß Fig. 1 mit runder Lochung unter der Nachweisschicht.

Fig. 3 zeigt einen Querschnitt durch einen erfindungsgemäßen diagnostischen Testträger gemäß Fig. 1 entlang A-A.

Fig. 4 zeigt eine Vergrößerung eines Teils des Querschnitts aus Fig. 3.

Fig. 5 zeigt eine perspektivische Ansicht eines erfindungsgemäßen diagnostischen Testträgers mit zwei Probenauftragsstellen.

Fig. 6 zeigt eine Aufsicht auf die Unterseite eines erfindungsgemäßen diagnostischen Testträgers gemäß Fig. 5 mit einer Lochung bestehend aus rundem und rechteckigem Loch unter 2 getrennten Nachweisschichten.

Fig. 7 zeigt einen Querschnitt durch einen erfindungsgemäßen diagnostischen Testträger gemäß Fig. 5 entlang A-A.

Fig. 8 zeigt eine perspektivische Ansicht eines erfindungsgemäßen diagnostischen Testträgers mit einer extra großen Probenauftragsstelle.

Fig. 9 zeigt eine Aufsicht auf die Unterseite eines erfindungsgemäßen diagnostischen Testträgers gemäß Fig. 8 mit einer Lochung bestehend aus rundem und rechteckigem Loch unter einer extra großen Nachweisschicht.

Fig. 10 zeigt einen Querschnitt durch einen erfindungsgemäßen diagnostischen Testträger gemäß Fig. 8 entlang A-A.

Fig. 11 zeigt eine perspektivische Ansicht eines erfindungsgemäßen diagnostischen Testträgers mit einer Probenauftragsstelle über einer von zwei Nachweisschichten.

Fig. 12 zeigt eine Aufsicht auf die Unterseite eines erfindungsgemäßen diagnostischen Testträgers gemäß Fig. 11 mit einer Lochung bestehend aus rundem und rechteckigem Loch unter zwei getrennten Nachweisschichten.

Fig. 13 zeigt einen Querschnitt durch einen erfindungsgemäßen diagnostischen Testträger gemäß Fig. 11 entlang A-A.

Fig. 14 zeigt eine perspektivische Ansicht eines erfindungsgemäßen diagnostischen Testträgers mit einer extra großen Probenauftragsstelle.

Fig. 15 zeigt eine Aufsicht auf die Unterseite eines erfindungsgemäßen diagnostischen Testträgers gemäß Fig. 14 mit einer Lochung bestehend aus einem extra großen rechteckigen Loch unter einer Nachweisschicht mit zwei aneinander angrenzenden Reaktionsbezirken.

Fig. 16 zeigt einen Querschnitt durch einen erfindungsgemäßen diagnostischen Testträger gemäß Fig. 14 entlang A-A.

Fig. 17 zeigt eine perspektivische Ansicht eines erfindungsgemäßen diagnostischen Testträgers mit einer Probenauftragsstelle über einer der beiden Reaktionsbezirke.

Fig. 18 zeigt eine Aufsicht auf die Unterseite eines erfindungsgemäßen diagnostischen Testträgers gemäß Fig. 17 mit einer Lochung bestehend aus einem extra großen rechteckigen Loch unter einer Nachweisschicht mit zwei aneinander angrenzenden Reaktionsbezirken.

Fig. 19 zeigt einen Querschnitt durch einen erfindungsgemäßen diagnostischen Testträger gemäß Fig. 17 entlang A-A.

Fig. 20 - 23 zeigen Kalibrationskurven 1 - 4 deren Erzeugung in Beispiel 2 beschrieben wird.

Die in den Figuren verwendeten Bezugszeichen bedeuten:
- 1: Diagnostischer Testträger
- 2: Tragschicht
- 3: Nachweisschicht
- 4: Netzwerk
- 5: Abdeckung
- 6: über die Nachweisschicht hinausragender Bereich des Netzwerkes
- 7: Probenauftragsstelle
- 8: Lochung
- 9: Reaktionsbezirk
- 10: Abstandhalter
- 11: kapillaraktiver Spalt
- 12: Probenflüssigkeit
- 13: Positionierloch
- 14: Klebebandbefestigung für Nachweisschicht

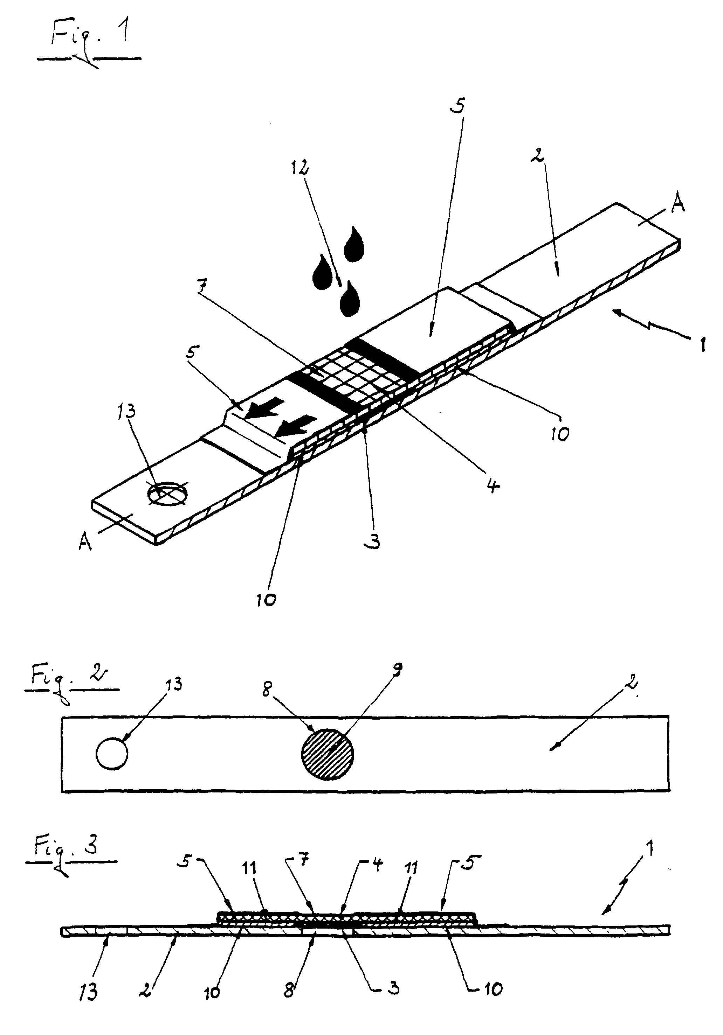

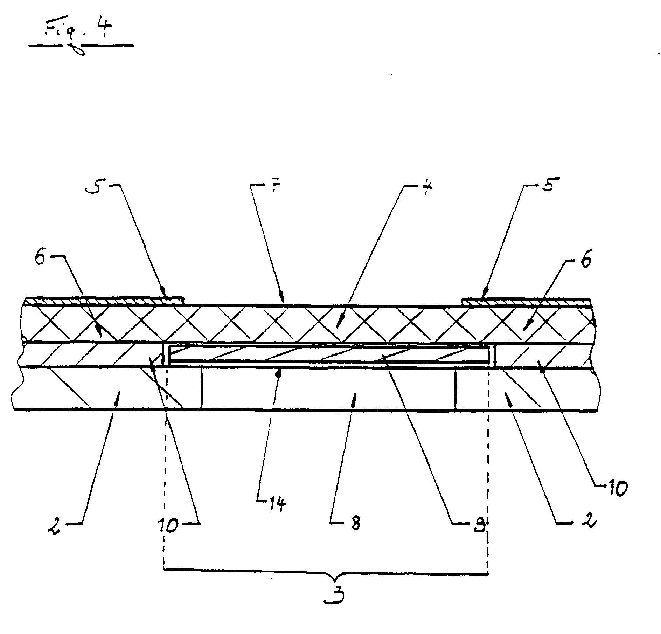

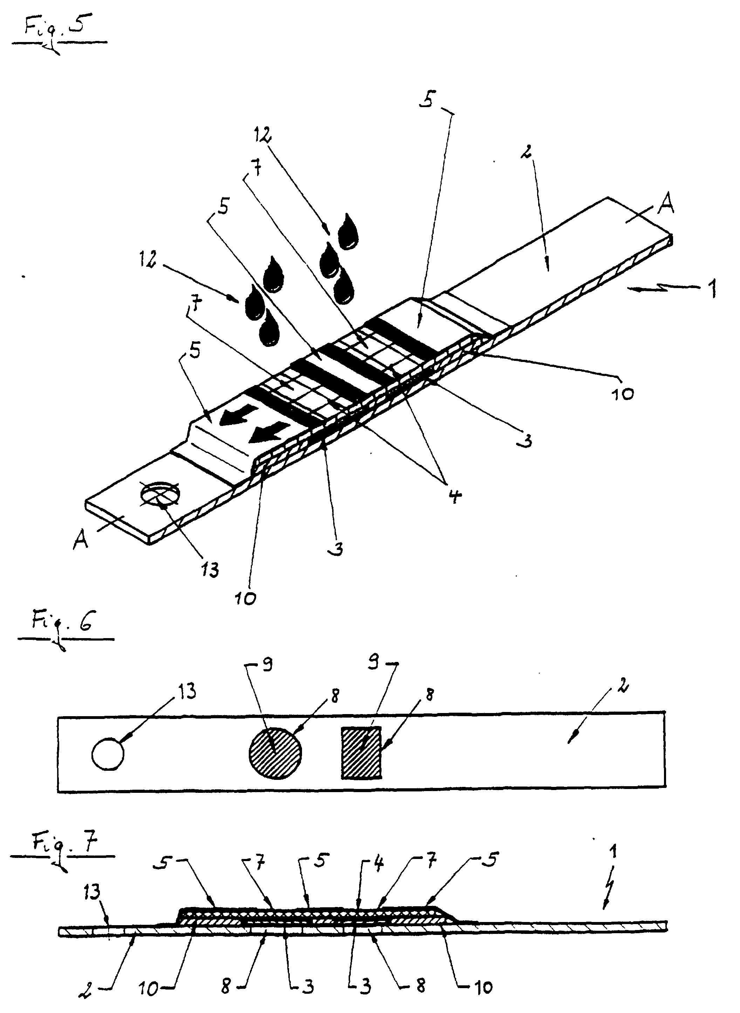

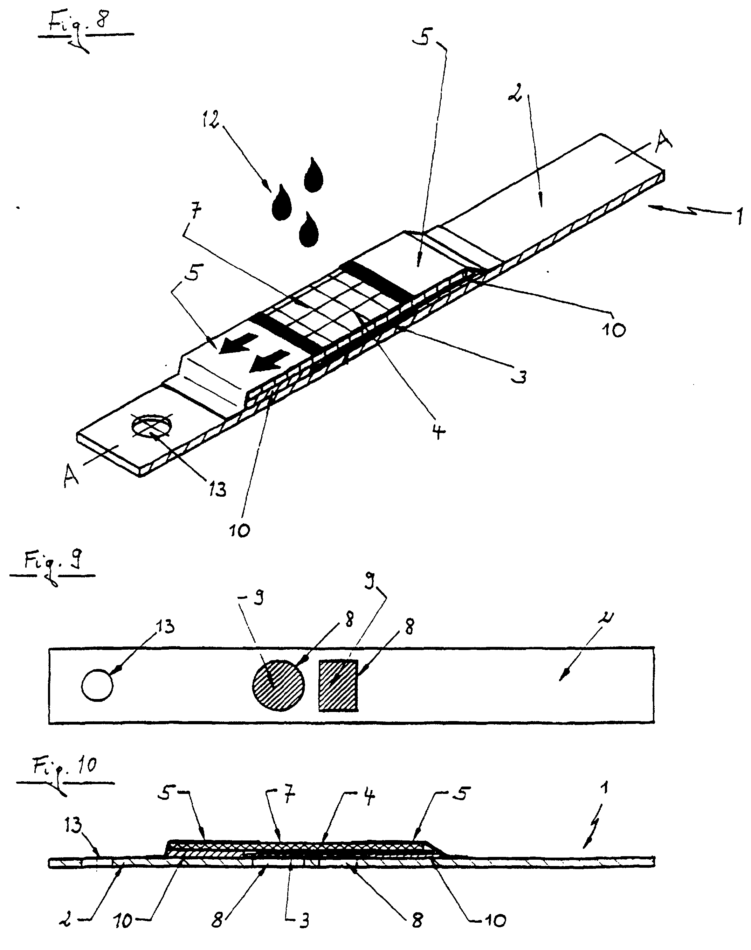

Der in Fig. 1 perspektivisch und in Fig. 3 im Querschnitt gezeigte erfindungsgemäße diagnostische Testträger (1) besitzt die Form eines Teststreifens. Auf einer Tragschicht (2) befindet sich eine Nachweisschicht (3), die durch ein größeres Netzwerk (4) überdeckt ist. Neben der Nachweisschicht (3) ist das Netzwerk (4) mittels Abstandhalter (10) auf der Tragschicht (2) befestigt. Diese Abstandhalter können Schmelzkleberflächen oder zweiseitig klebende Bänder sein, die das Netzwerk (4) auf der Tragschicht (2) fixieren. Idealerweise besitzen die Abstandhalter (10) in etwa die gleiche Dicke wie die Nachweisschicht (3). Die als Abdeckung (5) dienenden Schichten sind auf der Tragschicht (2) und dem Netzwerk (4) befestigt. Sie sind so angeordnet, daß sie den über die Nachweisschicht (3) hinausragenden Bereich des Netzwerkes (4) überdecken. Geringfügig ragen die Abdeckungen (5) auch noch über die Nachweisschicht (3). Sie lassen jedoch den größten Teil des Netzwerkes (4) frei, der die Nachweisschicht (3) überdeckt. Dieser Bereich stellt die Probenauftragsstelle (7) dar. Hierauf wird die zu untersuchende Probenflüssigkeit (12) aufgegeben. Das Positionierloch (13) dient dazu, den Teststreifen im Falle einer apparativen, beispielsweise einer reflektionsphotometrischen Vermessung an einer genau vorbestimmten Stelle des Apparates festzuhalten. Dies kann dadurch geschehen, daß beispielsweise ein Stift in das Positionierloch (13) hineinragt und so den Testträger (1) an einer vorbestimmten Stelle festhält. Die linke Abdeckung (5) enthält aufgedruckt Pfeile, die dem Anwender zeigen, mit welchem Ende der Testträger (1) in ein Meßgerät gelegt oder geschoben werden soll.

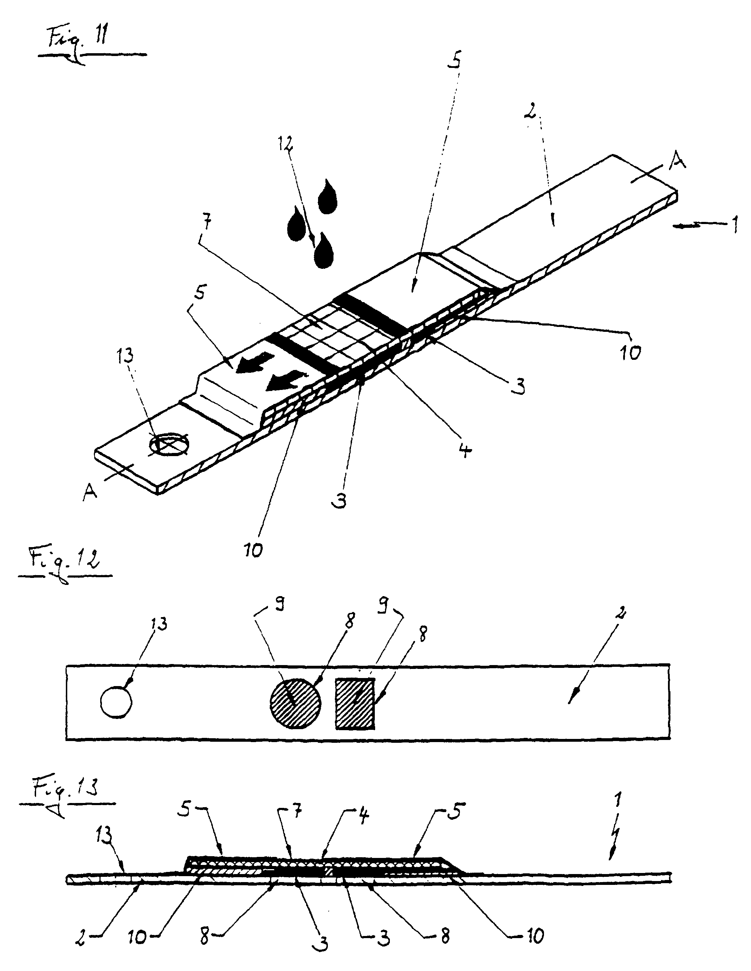

Fig. 4 zeigt einen vergrößerten Querschnitt durch einen erfindungsgemäßen diagnostischen Testträger, wie er in Fig. 1 und 3 dargestellt ist. An dieser Figur soll erläutert werden, wie ein Verfahren zur Bestimmung eines Analyts in einer flüssigen Probe abläuft. Für eine solche Bestimmung wird Probenflüssigkeit auf die Probenauftragsstelle (7) des Netzwerkes (4) gegeben. Die Flüssigkeit dringt vertikal durch das Netzwerk (4) in die Nachweisschicht (3), die ihrerseits mit doppelseitigem Klebeband (14) auf der Tragschicht (2) befestigt ist. Die Klebebandbefestigung (14) enthält ein Loch, das der Lochung (8) der Tragschicht (2) entspricht und das auch genau über dieser Lochung (8) liegt. Sofern ausreichend Probenflüssigkeit aufgegeben wurde, verteilt sich diese Flüssigkeit in der Nachweisschicht (3) über den gesamten Reaktionsbezirk (9). Bei sehr kleinen Flüssigkeitsvolumina kann die Nachweisschicht (3) das darüber liegende Netzwerk (4) sogar trocken saugen, da das Netzwerk (4) nicht selbst kapillaraktiv ist. Bei mittleren bis großen Flüssigkeitsvolumina füllen sich zunächst die Hohlräume des Netzwerkes (4) über der Nachweisschicht (3) und anschließend die kapillaren Hohlräume unter den Abdeckungen (5). Für die ordnungsgemäße Funktion dieser kapillaren Hohlräume ist es notwendig, daß die Abdeckungen (5) wenigstens etwas mit dem Bereich der Nachweisschicht (3) unter dem Netzwerk (4) überlappen. Durch die Lochung (8) kann der Reaktionsbezirk (9) der Nachweisschicht (3) beobachtet werden. Für diesen Aspekt ist in Fig. 2 eine Aufsicht auf die Unterseite des diagnostischen Testträgers gemäß Fig. 1, 3 und 4 dargestellt. Im Falle der Anwesenheit von Analyt in der aufgebrachten Probenflüssigkeit wird sich der Reaktionsbezirk (9) verändern. Es bildet sich ein Signal, beispielsweise eine Farbveränderung, deren Intensität ein Maß für die Menge an Analyt in der Probenflüssigkeit ist.

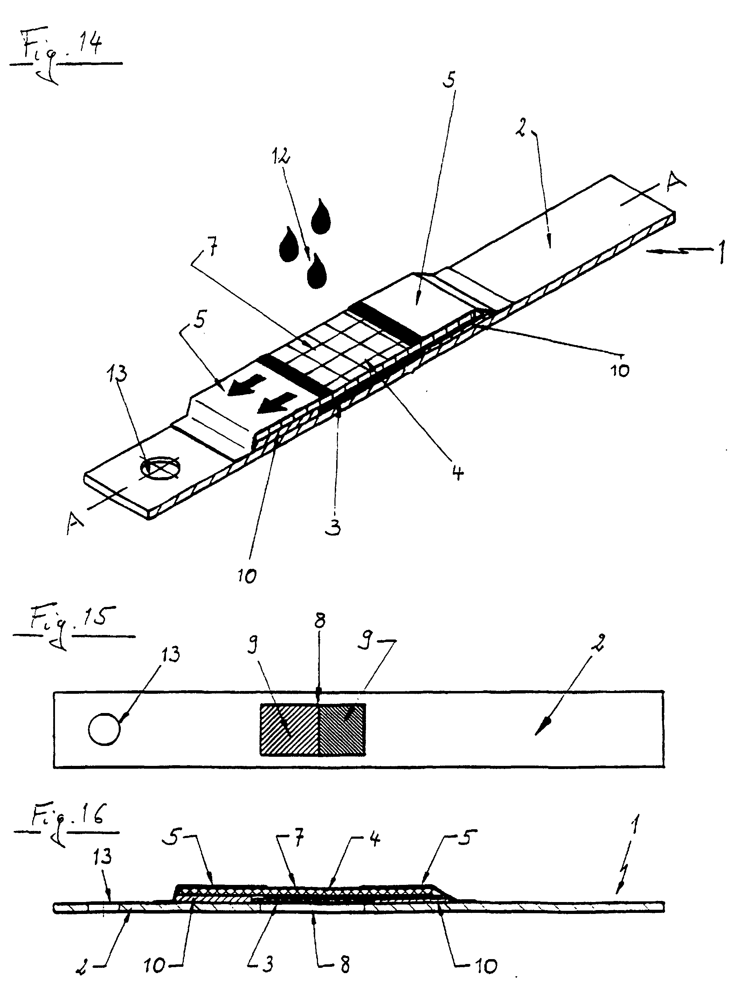

Bei dem in Fig. 5 bis 7 dargestellten erfindungsgemäßen diagnostischen Testträger handelt es sich um einen solchen mit zwei Nachweisschichten (3), die durch zwei darüber angeordnete Probenauftragsstellen (7) für Probenflüssigkeit (12) zugänglich sind. Die Probenauftragsstellen (7) werden durch drei bandförmige Abdeckungen (5) gebildet, die die Bereiche des Netzwerkes (4) überdecken, die über die Nachweisschichten (3) hinausragen. In dem abgebildeten Beispiel ist ein durchgehendes Netzwerk (4) verwendet worden. Es können aber auch zwei getrennte Netzwerke (4) mit einer dazwischenliegenden Flüssigkeitssperre, wie beispielsweise einem Klebeband oder einem Schmelzkleberstrich, verwendet werden. In der Tragschicht (2) des Testträgers (1) befindet sich eine Lochung (8) aus zwei Löchern, die jeweils einen Reaktionsbezirk (9) einer der beiden Nachweisschichten (3) beobachten lassen. Ein solcher Testträger (1) ist beispielsweise geeignet fiir die gleichzeitige Bestimmung zweier unterschiedlicher Analyten. Dabei ist die räumliche Trennung der Nachweisschichten (3) vorteilhaft, wenn sich die Reagenzien oder Reaktionsprodukte gegenseitig stören können.

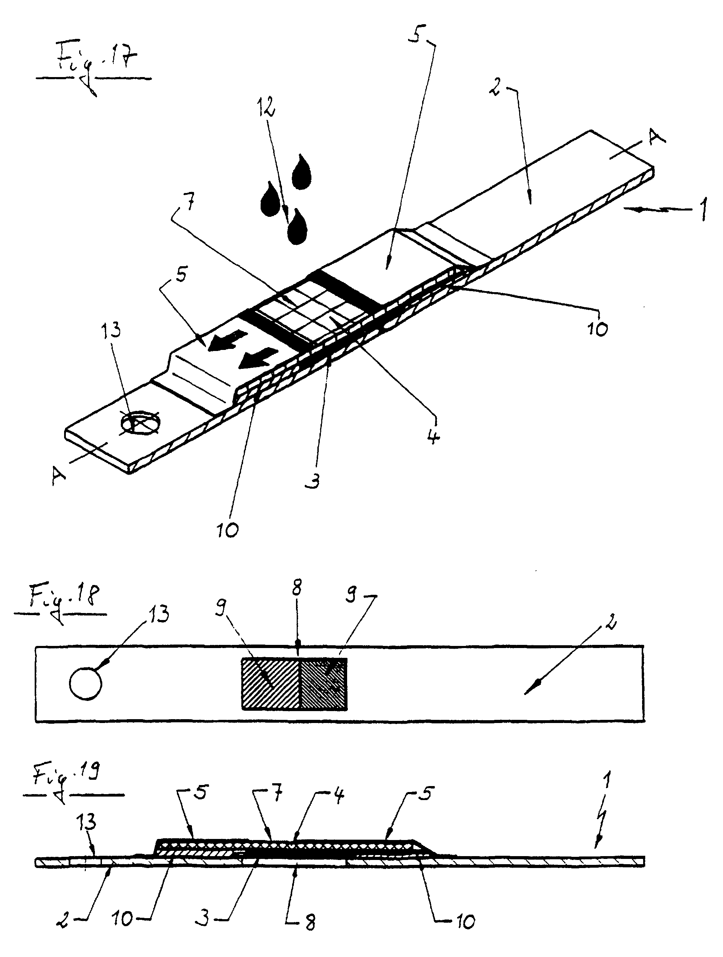

Der erfindungsgemäße diagnostische Testträger (1) gemäß Fig. 8 bis 10 besitzt eine extra große Probenauftragsstelle (7) über einer Nachweisschicht (3), die durch eine Lochung (8) aus zwei Löchern beobachtbar ist. Über beiden Löchern können beispielsweise unterschiedliche Reaktionsbezirke (9) angeordnet sein, die Reagenzien für unterschiedliche Analyte beinhalten. So können aus einer Probe zwei Analyte bestimmt werden. Die beiden Reaktionsbezirke können aber auch für die Bestimmung des gleichen Analyts mit unterschiedlicher Empfindlichkeit verwendet werden.

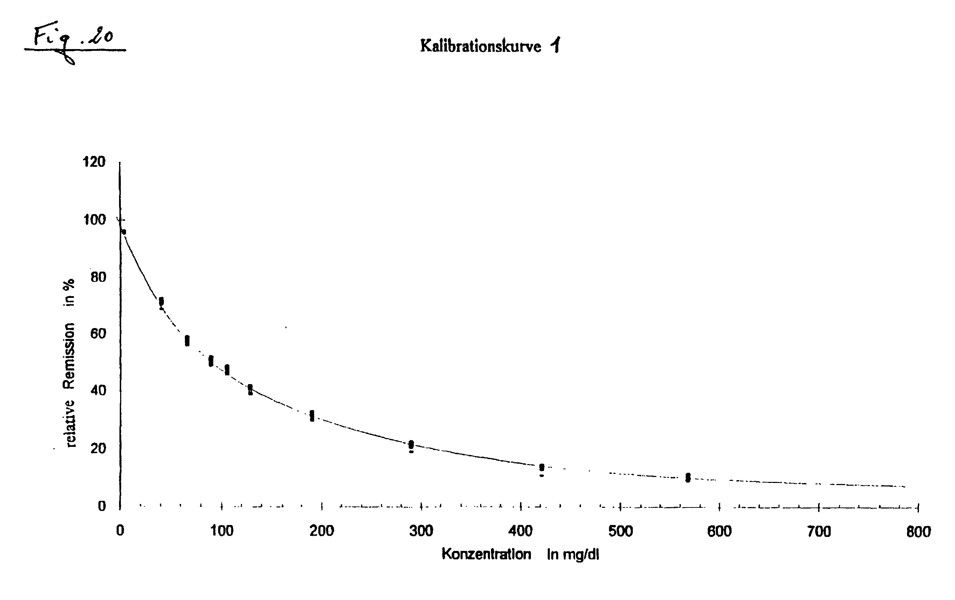

In Fig. 11 bis 13 ist ein erfindungsgemäßer diagnostischer Testträger (1) dargestellt, bei dem sich über einer Lochung (8) aus zwei Löchern zwei Nachweisschichten (3) befinden. Je eine Nachweisschicht (3) befindet sich über einem Loch der Lochung (8). Die Probenauftragsstelle (7) befindet sich in diesem Fall nur über einer der beiden Nachweisschichten (3). So gelangt Probenflüssigkeit (12) erst in die unter der Probenauftragsstelle (7) befindliche Nachweisschicht (3), bevor überschüssige Flüssigkeit durch Kapillarkräfte im Bereich des Netzwerkes (4) unterhalb der rechten Abdeckung (5) auch in die rechte Nachweisschicht (3), die durch das rechteckige Loch in der Tragfolie (2) beobachtet werden kann, gelangt. Beispielsweise ist ein solcher Testträger geeignet für die Bestimmung eines Analyten mit zwei unterschiedlich empfindlichen Nachweisschichten (3). Vorteilhafterweise befindet sich ein weniger empfindliches Universalfeld direkt unter der Probenauftragsstelle und ein zusätzliches hochempfindliches Feld daneben. Mit diesem Testträger ist bei kleinem Probevolumen eine Messung mit dem Universalfeld und bei großem Probevolumen eine verbesserte Messung mit beiden Feldern möglich.

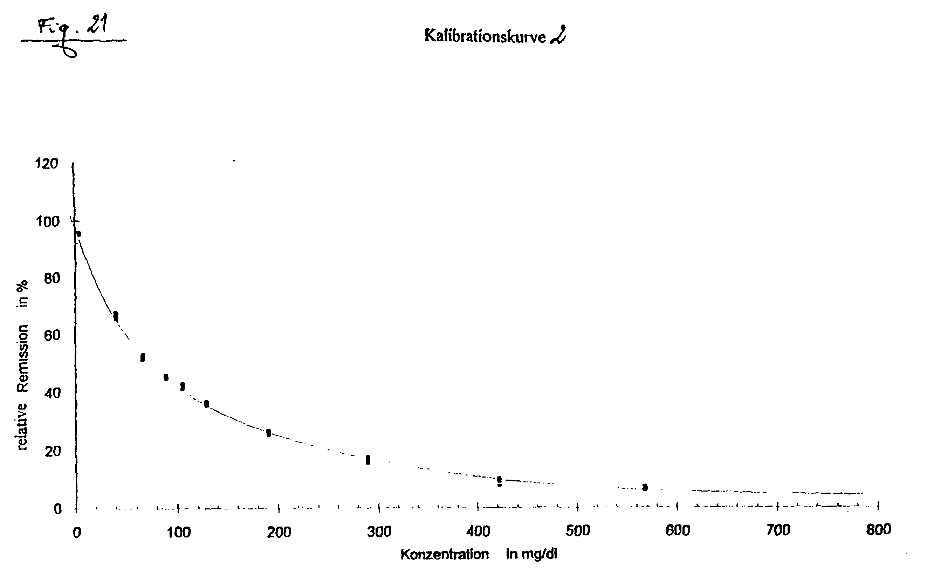

Der Testträger (1) gemäß Fig. 14-16 besitzt eine extra große Probenauftragsstelle (7) über einer Nachweisschicht (3), die zwei Reaktionsbezirke (9) trägt, die einander direkt benachbart sind. Diese beiden Reaktionsbezirke sind von der Unterseite der Tragschicht (2) durch die Lochung (8), die in diesem Fall aus nur einem einzigen rechteckigen Loch besteht, sichtbar. Probenflüssigkeit (12), die zentral auf die Probenauftragsstelle (7) aufgegeben wird, dringt durch das Netzwerk (4) in die Nachweisschicht (3) ein und erreicht gleichzeitig beide Reaktionsbezirke (9). Mit einem solchen Testträger können beispielsweise aus einer Probe zwei unterschiedliche Analyte bestimmt werden.

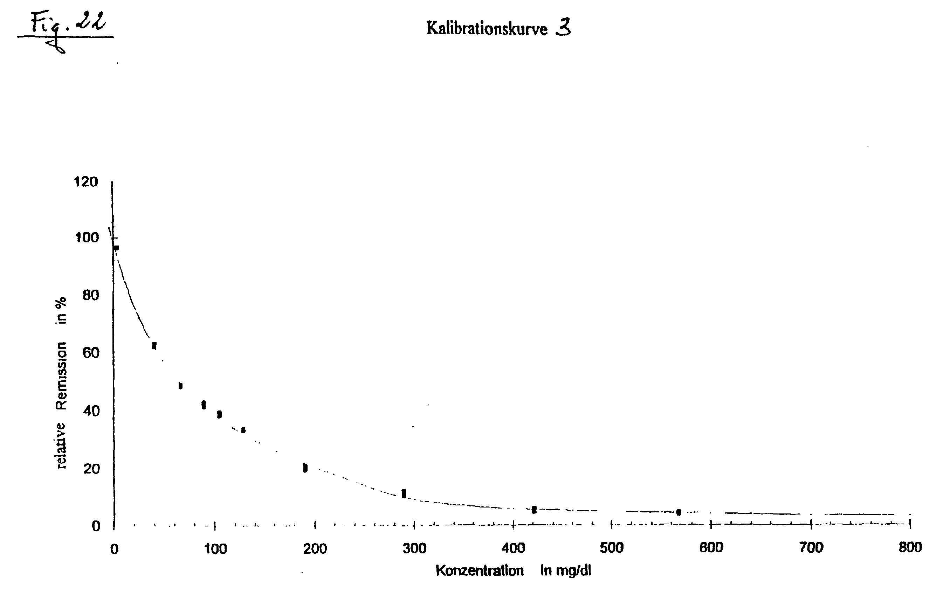

Der Testträger (1), der in Fig. 17-19 dargestellt ist, entspricht im wesentlichen dem Testträger gemäß Fig. 14-16. Jedoch befindet sich nun die Probenauftragsstelle (7) nur über einem der beiden Reaktionsbezirke (9). Der rechte Reaktionsbezirk (9) ist durch die rechte Abdeckung (5) vor der direkten Aufgabe von Probenflüssigkeit (12) geschützt. Hierher kann Probenflüssigkeit (12) nur über Kapillarkräfte innerhalb des Bereiches des Netzwerkes (4), der sich unter der rechten Abdeckung befindet, gelangen.

Die Erfindung wird durch die nachfolgenden Beispiele noch weiter erläutert.

### Beispiel 1

### Herstellung eines erfindungsgemäßen diagnostischen Testträgers fiir die Bestimmung von Glucose

Die Herstellung eines Testträgers gemäß Fig. 1 erfolgt mit folgenden Arbeitsschritten:

Auf eine Titandioxid-haltige Polyester-Tragschicht wird ein 5 mm breites doppelseitiges Klebeband (Polyesterträger und Synthesekautschuk-Kleber) aufgebracht. Dieser Verbund wird mit einem Lochabstand von 6 mm gemeinsam gestanzt, um die Meßlöcher zu erzeugen. Danach wird das Schutzpapier des doppelseitigen Klebebands abgezogen.

Zur Herstellung einer Nachweisschicht, die aus 2 Filmschichten aufgebaut ist, wird so vorgegangen:
A. In einem Becherglas werden die folgenden Komponenten als Reinsubstanzen oder in Form von Stammlösungen in folgender Zusammensetzung zusammengegeben und durch Rühren gemischt:

| | |
|---|---|
| Wasser | 820,0 g |
| Citronensäure-1-hydrat | 2,5 g |
| Calciumchlorid-2-hydrat | 0,5 g |
| Natriumhydroxid | 1,4 g |
| Xanthan gum | 3,4 g |
| Tetraethylammoniumchlorid | 2,0 g |
| N-Octanoyl-N-methyl-glucamid | 2,1 g |
| Polyvinylpyrrolidon (MG 25000) | 3,5 g |
| Transpafill® (Natrium-Aluminiumsilikat) | 62,1 g |
| Polyvinylpropionat-Dispersion (50 Gew.-% in Wasser) | 60,8 g |
| Bis-(2-hydroxyethyl)-(4-hydroximinocyclohexa-2,5-dienylidin)-ammoniumchlorid | 1,2 g |
| 2,18-Phosphormolybdänsäure-hexanatriumsalz | 16,1 g |
| Pyrrolochinolin-chinon | 32 mg |
| Glucosedehydrogenase rec. aus Acinetobacter calcoaceticus, | 1,7 MU |
| EC 1.1.99.17 | (2,4 g) |
| 1-Hexanol | 1,6 g |
| 1-Methoxy-2-propanol | 20,4 g |

Die Gesamtmasse wird mit NaOH auf einen pH von ca. 6 eingestellt und dann mit einem Flächengewicht von 89 g/qm auf eine 125 µ dicke Polycarbonatfolie aufgetragen und getrocknet.
B. In einem Becherglas werden die folgenden Komponenten als Reinsubstanzen oder in Form von Stammlösungen in folgender Zusammensetzung zusammengegeben und durch Rühren gemischt:

| | |
|---|---|
| Wasser | 579,7 g |
| Natriumhydroxid | 3,4 g |
| Gantrez® (Methylvinylether-maleinsäure-Copolymer) | 13,8 g |
| N-Octanoyl-N-methyl-glucamid | 3,6 g |
| Tetraethylammoniumchlorid | 9,7 g |
| Polyvinylpyrrolidon (MG 25000) | 20,2 g |
| Titandioxid | 177,1 g |
| Kieselgur | 55,3 g |
| Polyvinylpropionat-Dispersion (50 Gew.-% in Wasser) | 70,6 g |
| 2,18-Phosphormolybdänsäure-hexanatriumsalz | 44,3 g |
| Kaliumhexacyanoferrat (III) | 0,3 g |
| 1-Hexanol | 1,6 g |
| 1-Methoxy-2-propanol | 20,4 g |

Die Gesamtmasse wird mit NaOH auf einen pH von ca. 6 eingestellt und dann mit einem Flächengewicht von 104 g/qm auf die wie vorstehend unter A. beschriebene beschichtete Polycarbonatfolie aufgetragen und getrocknet.

Ein 5 mm breiter Streifen der so hergestellten Nachweisschicht wird mit der Folienseite auf das gestanzte doppelseitige Klebeband auf der Tragschicht passgenau aufgeklebt.

Direkt an die Nachweisschicht angrenzend werden auf beiden Seiten doppelseitige Klebebänder (PVC-Träger und Naturkautschuk-Kleber) als Abstandshalter auf die Trägerfolie aufgeklebt, Im vorliegenden Beispiel ist ein Abstandshalter 6 mm und der andere 9 mm breit. Danach wird die Schutzfolie der beiden doppelseitigen Klebebänder abgezogen.

Auf diesen Verbund wird ein mit Netzmittel imprägniertes gelbes monofiles grobmaschiges Polyestergewebe Scrynel PE 280 HC (Züricher Beuteltuchfabrik, Rüschlikon, Schweiz) aufgelegt und durch Anpressen verklebt.

Es werden zwei einseitige Klebebänder (PVC-Träger und Naturkautschuk-Kleber) als Abdeckungen so auf das gelbe Netz aufgeklebt, daß die Abstandshalter ganz abgedeckt werden und wenigstens noch eine geringfügige Überlappung mit dem Reaktivbezirk stattfindet. Damit ist die Bandware fertiggestellt.

Die Bandware wird so in 6 mm breite Testträger geschnitten, daß das Meßloch im Testträger mittig liegt.

### Beispiel 2

### Volumenunabhängigkeit der erfindungsgemäßen Testträger

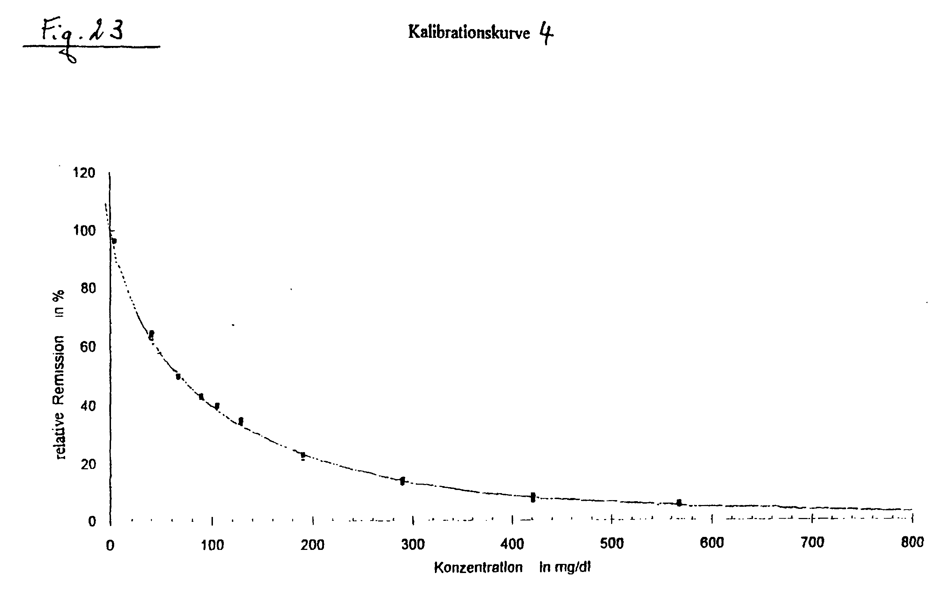

Die Testträger aus Beispiel 1 können mit einem Reflexionsphotometer vermessen werden. Die Remissionswerte, die ein Maß für die Farbintensität darstellen, können bei Vorliegen einer Kalibrationskurve in Glucosekonzentrationen umgerechnet werden. Sofern die Bezeichnung "relative Remissionen" verwendet wird, sind die Remissionen auf den trockenen Testträger bezogen.
A. Kalibrationskurven werden dadurch erzeugt, daß eine große Zahl von Venenbluten mit unterschiedlichen Glucosekonzentrationen vermessen wird. Aus den Remissionswerten und den mit einer Referenzmethode ermittelten Glucosekonzentrationen dieser Venenblute kann dann eine Kalibrationskurve erstellt werden.
Bei der Kalibrationsvariante 1 wurden 10 µl Venenblut auf Testträger gemäß Beispiel 1 aufgetragen und die Remissionen nach 21 sec erfaßt. Aus den gemittelten Remissionen von 10 Testträgern und den Referenzwerten der Blutproben wurde die Kalibrationskurve 1 (Fig. 20) durch Regressionsrechnung ermittelt.
Bei der Kalibrationsvariante 2 wurden ebenfalls 10 µl Venenblut auf Testträger gemäß Beispiel 1 aufgetragen und die Remissionen nach 30 sec erfaßt. Aus den gemittelten Remissionen von 10 Testträgern und den Referenzwerten der Blutproben wurde die Kalibrationskurve 2 (Fig. 21) durch Regressionsrechnung ermittelt.
Bei der Kalibrationsvariante 3 wurden ebenfalls 10 µl Venenblut auf Testträger gemäß Beispiel 1 aufgetragen und die Remissionen in Abständen von 3 sec erfaßt. Sobald die Remissionsdifferenzen zweimal hintereinander geringer als 0,3 waren, wurde die Messung abgebrochen und der Remissionswert zur Auswertung herangezogen. Aus den gemittelten Remissionen von 10 Testträgern und den Referenzwerten der Blutproben wurde die Kalibrationskurve 3 (Fig. 22) durch Regressionsrechnung ermittelt.
Bei der Kalibrationsvariante 4 wurden ebenfalls 10 µl Venenblut auf Testträger gemäß Beispiel 1 aufgetragen und die Remissionen in Abständen von 3 sec erfaßt. Sobald die Remissionsdifferenzen zweimal hintereinander geringer als 0,9 waren, wurde die Messung abgebrochen und der Remissionswert zur Auswertung herangezogen. Aus den gemittelten Remissionen von 10 Testträgern und den Referenzwerten der Blutproben wurde die Kalibrationskurve 4 (Fig. 23) durch Regressionsrechnung ermittelt.
B. Bei Meßvariante 1 wurden unterschiedliche Volumina von Venenblut auf Testträger gemaß Beispiel 1 aufgetragen und die Remissionen nach 21 sec erfaßt. Die einzelnen Remissionen wurden mit der entsprechenden Kalibrationskurve gemäß Fig. 20 in Glucosekonzentrationen umgerechnet. Aus den gemittelten Konzentrationen von 10 Testträgern und den Referenzwerten der Blutproben wurde die Richtigkeitsabweichung ermittelt und in Tabelle 1 dargestellt.
Bei der Meßvariante 2 wurden ebenfalls unterschiedliche Volumina von Venenblut auf Testträger gemäß Beispiel 1 aufgetragen und die Remissionen nach 30 sec erfaßt. Die einzelnen Remissionen wurden mit der entsprechenden Kalibrationskurve gemäß Fig. 21 in Glucosekonzentrationen umgerechnet. Aus den gemittelten Konzentrationen von 10 Testträgern und den Referenzwerten der Blutproben wurde die Richtigkeitsabweichung ermittelt und in Tabelle 2 dargestellt.
Bei der Meßvariante 3 wurden ebenfalls unterschiedliche Volumina von Venenblut auf Testträger gemäß Beispiel 1 aufgetragen und die Remissionen in Abständen von 3 sec erfaßt. Sobald die Remissionsdifferenzen zweimal hintereinander geringer als 0,3 waren, wurde die Messung abgebrochen und der Remissionswert zur Auswertung herangezogen. Die einzelnen Remissionen wurden mit der entsprechenden Kalibrationskurve gemäß Fig. 22 in Glucosekonzentrationen umgerechnet. Aus den gemittelten Konzentrationen von 10 Testträgern und den Referenzwerten der Blutproben wurde die Richtigkeitsabweichung ermittelt und in Tabelle 3 dargestellt.
Bei der Meßvariante 4 wurden ebenfalls unterschiedliche Volumina von Venenblut auf Testträger gemäß Beispiel 1 aufgetragen und die Remissionen in Abständen von 3 sec erfaßt. Sobald die Remissionsdifferenzen zweimal hintereinander geringer als 0,9 waren, wurde die Messung abgebrochen und der Remissionswert zur Auswertung herangezogen. Die einzelnen Remissionen wurden mit der entsprechenden Kalibrationskurve gemäß Fig. 23 in Glucosekonzentrationen umgerechnet. Aus den gemittelten Konzentrationen von 10 Testträgern und den Referenzwerten der Blutproben wurde die Richtigkeitsabweichung ermittelt und in Tabelle 4 dargestellt.

**Tabelle 1:**

| Volumentoleranz des Teststreifens bei Meßvariante 1 | | | |
|---|---|---|---|
| Probevolumen | gemessene relative Remission [%] | berechnete Konzentration nach Kalibrationskurve 1 | prozentuale Abweichung vom Referenzwert |
| 3 µl | 42,8 | 117,5 | -0,5 |
| 5 µl | 42.9 | 117,1 | -0,8 |
| 8 µl | 42,6 | 118,5 | 0,4 |
| 10 µl | 41,8 | 122,1 | 3,4 |
| 20 µl | 41,9 | 121,6 | 3,0 |

**Tabelle 2:**

| Volumentoleranz des Teststreifens bei Meßvariante 2 | | | |
|---|---|---|---|
| Probevolumen | gemessene relative Remission [%] | berechnete Konzentration nach Kalibrationskurve 2 | prozentuale Abweichung vom Referenzwert |
| 3 µl | 37,4 | 117,5 | -0,5 |
| 5 µl | 37,6 | 117,0 | -0,9 |
| 8 µl | 37,4 | 117,7 | -0,3 |
| 10 µl | 37,2 | 118,6 | 0,4 |
| 20 µl | 37,0 | 119,4 | 1,1 |

**Tabelle 3:**

| Volumentoleranz des Teststreifens bei Meßvariante 3 | | | |
|---|---|---|---|
| Probevolumen | gemessene relative Remission [%] | berechnete Konzentration nach Kalibrationskurve 3 | prozentuale Abweichung vom Referenzwert |
| 3 µl | 33,4 | 120,2 | 1,5 |
| 5 µl | 34,0 | 117,7 | - 0,6 |
| 8 µl | 33,9 | 118,0 | -0,3 |
| 10 µl | 34,1 | 117,0 | - 1,2 |
| 20 µl | 33,8 | 118,5 | 0,1 |

**Tabelle 4:**

| Volumentoleranz des Teststreifens bei Meßvariante 4 | | | |
|---|---|---|---|
| Probevolumen | gemessene relative Remission [%] | berechnete Konzentration nach Kalibrationskurve 4 | prozentuale Abweichung vom Referenzwert |
| 3 µl | 35,2 | 119,2 | 0,8 |
| 5 µl | 35,3 | 118,7 | 0,3 |
| 8 µl | 35,6 | 117,3 | -0,8 |
| 10 µl | 35,6 | 117,4 | -0,8 |
| 20 µl | 35,4 | 118,0 | - 0,3 |

C. Wie aus den Tabellen ersichtlich sind die erfindungsgemäßen Testträger weitgehend volumenunabhängig.

## Patentansprüche

1. Diagnostischer Testträger (1) enthaltend eine Tragschicht (2) mit einer darauf angeordneten zur Bestimmung von Analyt in flüssiger Probe erforderliche Reagenzien enthaltenden Nachweisschicht (3) und ein die Nachweisschicht (3) überdeckendes Netzwerk (4), das größer als die Nachweisschicht (3) ist und das auf der Tragschicht (2) befestigt ist, **dadurch gekennzeichnet, daß** das Netzwerk (4) hydrophil aber alleine nicht kapillaraktiv ist, und eine inerte Abdeckung (5) aus probenundurchlässigem Material über den über die Nachweisschicht hinausragenden Bereichen (6) des Netzwerkes so angeordnet ist, daß eine Probenauftragsstelle (7) auf dem die Nachweisschicht überdeckenden Bereich des Netzwerkes (4) frei bleibt und das Netzwerk (4) zwischen Abdeckung (5) und Nachweisschicht (3) sowie zwischen Abdeckung (5) und Tragschicht (2) beziehungsweise zwischen Abdeckung (5) und Abstandhalter (10) auf der Tragschicht (2) einen kapillaraktiven Spalt (11) erzeugt.

2. Diagnostischer Testträger gemäß Patentanspruch 1, **dadurch gekennzeichnet, daß** auf der Tragschicht mehrere Nachweisschichten nebeneinander angeordnet sind.

3. Diagnostischer Testträger gemäß Patentansprüchen 1 oder 2, **dadurch gekennzeichnet, daß** die Tragschicht gelocht und die Nachweisschicht(en) über der Lochung angeordnet ist (sind).

4. Diagnostischer Testträger gemäß Patentanspruch 3, **dadurch gekennzeichnet, daß** sich die Probenauftragsstelle über der Lochung der Tragschicht befindet.

5. Diagnostischer Testträger gemäß Patentanspruch 3, **dadurch gekennzeichnet, daß** sich die Probenauftragsstelle nicht über der Lochung der Tragschicht befindet.

6. Diagnostischer Testträger gemäß einem der vorangehenden Patentansprüche, **dadurch gekennzeichnet, daß** die Tragschicht als Lochung mehrere Löcher enthält über denen eine oder mehrere Nachweisschichten angeordnet sind.

7. Diagnostischer Testträger gemäß einem der Patentansprüche 2-5, **dadurch gekennzeichnet, daß** die Tragschicht als Lochung mehrere Löcher enthält, über denen jeweils unterschiedliche Nachweisschichten angeordnet sind.

8. Diagnostischer Testträger gemäß Patentanspruch 1, **dadurch gekennzeichnet, daß** die Tragschicht ein Loch enthält, über dem eine Nachweisschicht mit mehreren nebeneinander befindlichen Reaktionsbezirken angeordnet ist.

9. Diagnostischer Testträger gemäß einem der Patentansprüche 7 oder 8, **dadurch gekennzeichnet, daß** sich über mehreren oder allen Nachweisschichten oder Reaktionsbezirken jeweils eine Probenauftragsstelle befindet.

10. Diagnostischer Testtrager gemäß einem der Patentansprüche 7 oder 8, **dadurch gekennzeichnet, daß** sich nur über einer der Nachweisschichten oder einem Reaktionsbezirk eine Probenauftragsstelle befindet.

11. Diagnostischer Testträger gemäß einem der vorangehenden Patentansprüche, **dadurch gekennzeichnet, daß** das Netzwerk ein monofiles Gewebe ist.

12. Diagnostischer Testträger gemäß einem der vorangehenden Patentansprüche, **dadurch gekennzeichnet, daß** das Netzwerk mittels eines Klebebandes, bevorzugt mit Natur- oder Synthesekautschuk, auf der Tragschicht befestigt ist.

13. Verwendung eines diagnostischen Testträgers gemäß einem der Patentansprüche 1-12 zur Bestimmung von Analyt in einer Flüssigkeit.

14. Verfahren zur Bestimmung von Analyt in flüssiger Probe mit Hilfe eines diagnostischen Testträgers gemäß einem der Patentansprüche 1-12, wobei Probenflüssigkeit auf die Probenauftragsstelle aufgegeben wird, überschüssige Flüssigkeit, die nicht von der (den) Nachweisschicht(en) und dem (den) darüber liegenden Bereich(en) des Netzwerkes (4) aufgenommen wird, in den über die Nachweisschicht(en) hinausragenden Bereich des Netzwerkes weggeführt wird und die Nachweisschicht(en) auf eine Signalbildung hin beobachtet wird (werden), wobei die Signalbildung ein Maß fiir die Anwesenheit bzw. Menge an Analyt in der untersuchten flüssigen Probe darstellt.

## Claims

1. Diagnostic test carrier (1) comprising a supporting layer (2) with a detection layer (3) arranged thereon containing the reagents required to determine analyte in a liquid sample and a network (4) covering the detection layer (3) which is larger than the detection layer (3) and which is attached to the supporting layer (2), wherein the network (4) is hydrophilic but not capillary active on its own and an inert cover (5) made of sample-impermeable material is arranged over the areas (6) of the network that extend beyond the detection layer in such a way that a sample application site (7) remains free on the region of the network (4) that covers the detection layer and the network (4) produces a capillary active gap (11) between the cover (5) and the detection layer (3) as well as between the cover (5) and the supporting layer (2) or between the cover (5) and the spacers (10) on the supporting layer (2).

2. Diagnostic test carrier as claimed in claim 1, wherein several detection layers are arranged next to one another on the supporting layer.

3. Diagnostic test carrier as claimed in claims 1 or 2, wherein the supporting layer is perforated and the detection layer(s) is(are) arranged over the perforation.

4. Diagnostic test carrier as claimed in claim 3, wherein the sample application site is located over the perforation of the supporting layer.

5. Diagnostic test carrier as claimed in claim 3, wherein the sample application site is not located over the perforation of the supporting layer.

6. Diagnostic test carrier as claimed in one of the previous claims, wherein the supporting layer contains several holes as the perforation over which one or several detection layers are arranged.

7. Diagnostic test carrier as claimed in one of the claims 2-5, wherein the supporting layer contains several holes as the perforation over each of which different detection layers are located.

8. Diagnostic test carrier as claimed in claim 1, wherein the supporting layer contains a hole over which a detection layer containing several adjacent reaction zones is arranged.

9. Diagnostic test carrier as claimed in one of the claims 7 or 8, wherein a sample application site is located in each case over several or all detection layers or reaction zones.

10. Diagnostic test carrier as claimed in one of the claims 7 or 8, wherein a sample application site is located only over one of the detection layers or one reaction zone.

11. Diagnostic test carrier as claimed in one of the previous claims, wherein the network is a monofilament fabric.

12. Diagnostic test carrier as claimed in one of the previous claims, wherein the network is attached to the supporting layer by means of an adhesive tape preferably containing natural or synthetic rubber.

13. Use of a diagnostic test carrier as claimed in one of the claims 1-12 for the determination of analyte in a liquid.

14. Method for the determination of analyte in a liquid sample with the aid of a test carrier as claimed in one of the claims 1-12, wherein sample liquid is applied to the sample application site, excess liquid which is not taken up by the detection layer(s) and the region(s) of the network (4) located above it (them) is led away into the region of the network that extends beyond the detection layer(s) and the detection layer(s) is(are) observed for signal formation, the signal formation being a measure of the presence or amount of analyte in the examined liquid sample.

## Revendications

1. Support d'essai diagnostique (1) contenant une couche de support (2) sur laquelle est disposée une couche de décèlement (3) contenant des réactifs requis pour la détermination d'un analyte dans un échantillon liquide et une structure réticulée (4) recouvrant la couche de décèlement (3), qui est plus grande que la couche de décèlement (3) et qui est fixée sur la couche de support (2), **caractérisée en ce que** la structure réticulée (4) est hydrophile, mais en soi n'a pas d'activité capillaire, et un recouvrement inerte (5) constitué d'un matériau imperméable à l'échantillon est disposé par-dessus les zones (6) de la structure réticulée faisant saillie par rapport à la couche de décèlement, de telle sorte qu'un endroit d'application de l'échantillon (7) reste libre sur la zone de la structure réticulée (4) recouvrant la couche de décèlement et de telle sorte que la structure réticulée (4) forme une fente à activité capillaire (11) entre le recouvrement (5) et la couche de décèlement (3) et entre le recouvrement (5) et la couche de support (2), respectivement entre le recouvrement (5) et la pièce d'écartement (10) sur la couche de support (2).

2. Support d'essai diagnostique selon la revendication 1, **caractérisé en ce que** plusieurs couches de décèlement sont disposées les unes à côté des autres sur la couche de support.

3. Support d'essai diagnostique selon la revendication 1 ou 2, **caractérisé en ce que** la couche de support est perforée et la ou les couches de décèlement est ou sont disposées par-dessus la perforation.

4. Support d'essai diagnostique selon la revendication 3, **caractérisé en ce que** l'endroit d'application de l'échantillon est disposé par-dessus la perforation de la couche de support.

5. Support d'essai diagnostique selon la revendication 3, **caractérisé en ce que** l'endroit d'application de l'échantillon n'est pas disposé par-dessus la perforation de la couche de support.

6. Support d'essai diagnostique selon l'une quelconque des revendications précédentes, **caractérisé en ce que** la couche de support contient à titre de perforation, plusieurs trous, par-dessus lesquels sont disposées une ou plusieurs couches de décèlement.

7. Support d'essai diagnostique selon l'une quelconque des revendications 2 à 5, **caractérisé en ce que** la couche de support contient, à titre de perforation, plusieurs trous par-dessus lesquels sont disposées respectivement des couches de décèlement différentes.

8. Support d'essai diagnostique selon la revendication 1, **caractérisé en ce que** la couche de support contient un trou par-dessus lequel est disposée une couche de décèlement comprenant plusieurs zones réactionnelles disposées les unes à côté des autres.

9. Support d'essai diagnostique selon l'une quelconque des revendications 7 ou 8, **caractérisé en ce qu'**un endroit d'application de l'échantillon est disposé respectivement par-dessus plusieurs couches de décèlement ou plusieurs zones réactionnelles ou par-dessus l'ensemble desdites couches ou desdites zones.

10. Support d'essai diagnostique selon l'une quelconque des revendications 7 ou 8, **caractérisé en ce qu'**un endroit d'application de l'échantillon est disposé uniquement sur une des couches de décèlement ou sur uniquement une zone réactionnelle.

11. Support d'essai diagnostique selon l'une quelconque des revendications précédentes, **caractérisé en ce que** la structure réticulée est un tissu monofil.

12. Support d'essai diagnostique selon l'une quelconque des revendications précédentes, **caractérisé en ce que** la structure réticulée est fixée à l'aide d'une bande adhésive, de préférence avec du caoutchouc naturel ou du caoutchouc synthétique, sur la couche de support.

13. Utilisation d'un support d'essai diagnostique selon l'une quelconque des revendications 1 à 12 pour la détermination d'un analyte dans un liquide.

14. Procédé pour la détermination d'un analyte dans un échantillon liquide à l'aide d'un support d'essai diagnostique selon l'une quelconque des revendications 1 à 12, dans lequel un liquide d'échantillon est appliqué sur l'endroit d'application de l'échantillon, le liquide en excès, qui n'est pas absorbé par la couche ou les couches de décèlement et par la zone ou les zones de la structure réticulée (4) disposées par-dessus ladite ou lesdites couches, est évacué dans la zone de la structure réticulée faisant saillie par rapport à la couche ou aux couches de décèlement, et la couche ou les couches de décèlement est ou sont observées en ce qui concerne une signalisation, la signalisation représentant une mesure de la présence respectivement de la quantité d'un analyte dans l'échantillon liquide soumis à l'analyse.
